# EUROPÄISCHE PATENTSCHRIFT

(11) **EP 1 475 071 B1**
(45) Veröffentlichungstag und Bekanntmachung des Hinweises auf die Patenterteilung: **27.02.2008**
(21) Anmeldenummer: 03010468.1
(22) Anmeldetag: 09.05.2003
(51) Int. Cl.: A61Q 13/00, A61K 8/37, C11B 9/00

(54) **Kosmetische Zusammensetzungen enthaltend Cyclohexanpolycarbonsäurederivate**
Cosmetic compositions comprising cyclohexanepolycarboxylic acid derivatives
Compositions cosmétiques contenant des dérivés de l'acide cyclohexanepolycarboxylique

(43) Veröffentlichungstag der Anmeldung: 10.11.2004
(73) Patentinhaber: BASF Aktiengesellschaft, 67056 Ludwigshafen (DE)
(72) Erfinder: Storzum, Uwe, 67551 Worms (DE); Breitscheidel, Boris Dr., 36043 Fulda (DE)
(74) Vertreter: Isenbruck, Günter

(56) Entgegenhaltungen:
- EP-A- 0 373 838
- EP-A- 0 694 605
- WO-A-02/13776
- DE-C- 754 482
- US-A- 5 744 129
- US-A- 5 882 636
- US-A- 5 925 336
- US-A1- 2001 007 676
- DATABASE CA [Online] CHEMICAL ABSTRACTS SERVICE, COLUMBUS, OHIO, US; MARUZENI, SHOJI ET AL: "Perfumes containing cyclohexanedicarboxylic acid diesters" retrieved from STN Database accession no. 88:94683 XP002264591 & JP 52 136927 A (ASAHI DENKA KOGYO K. K., JAPAN) 16. November 1977 (1977-11-16)
- DATABASE CA [Online] CHEMICAL ABSTRACTS SERVICE, COLUMBUS, OHIO, US; ZHANG, YUMING ET AL: "Analysis of cis- and trans-isomers of perfumes by crosslinked glass capillary chromatographic column" retrieved from STN Database accession no. 107:161353 XP002264592 & SEPU (1987), 5(2), 108-10 ,

## Beschreibung

Die vorliegende Erfindung betrifft kosmetische Zusammensetzungen, insbesondere Riechstoffzusammensetzungen und kosmetische Zusammensetzungen zur dekorativen Anwendung am menschlichen Körper, enthaltend mindestens ein Cyclohexanpolycarbonsäurederivat, sowie die Verwendung von Cyclohexanpolycarbonsäurederivaten in kosmetischen Zusammensetzungen.

Kosmetische Zusammensetzungen jeglicher Art enthalten vielfach Polycarbonsäurederivate, insbesondere aromatische Polycarbonsäurederivate für verschiedene Anwendungszwecke.

Beispielsweise werden Polycarbonsäureester in Riechstoffzusammensetzungen eingesetzt.

EP-A-0 684 037 betrifft desodorierende Zusammensetzungen, die eine Riechstoffzusammensetzung und ein anorganisches desodorierendes Material enthalten. Die Riechstoffzusammensetzung enthält bevorzugt Ester, insbesondere Diethylphthalat.

WO 02/13776 betrifft ein Deodorant enthaltend aktives Deodorant, Geliermittel auf Basis von Seife, eine Riechstoffzusammensetzung, ein auf der Haut kühlendes Mittel, das in einem Lösungsmittelsystem enthaltend einen nicht-wässrigen, flüssigen Träger und Wasser, solubilisiert ist. Die Riechstoffzusammensetzung enthält Ester wie Diethylphthalat.

In EP-A-1 068 862 ist eine desodorierende Wirkstoffkombination enthaltend einen mehrwertigen Alkohol, insbesondere ein 1,2-Diol, und mindestens einen Zitronensäuretrialkylester offenbart.

EP-A-694605 offenbart eine Riechstoffzusammensetzung, die C₁-C₄ Cyclohexandicarbonsäureester enthält.

JP-A-52136927 beschreibt Cyclohexandcarbonsäureester als Duftstoffe. Die Isononyl-Verbindung wird nicht erwähnt.

Des weiteren ist der Einsatz von Polycarbonsäureestern in kosmetischen Zusammensetzungen zur dekorativen Anwendung am menschlichen Körper, zum Beispiel in Nagellacken und kosmetischen Stiften, bekannt.

So betrifft DE 200 09 445 U1 kosmetische Zubereitungen, bevorzugt in Form von Stiften wie Lippenstiften, Lippenpflegestiften, Abdeckstiften usw., wobei als Ölkomponente zum Beispiel Ester aus Alkandicarbonsäuren eingesetzt werden.

US 2001/000 7676 A1 betrifft Nagellackzusammensetzungen, die zum Beispiel Dibutylphthalat als Weichmacher enthalten.

Da Phthalate allergieauslösend wirken können, sind in US 5,882,636 Nagellackzusammensetzungen offenbart, die phthalatfrei sind und an Stelle von zum Beispiel Dibutylphthalat Adipate, Pentaerythiryltetrabenzoat, 2,2,4-Trimethyl-1,3-pentandioldiisobutyrat, Pentaerythiryltetraacetat und Mischungen davon enthalten.

Aufgabe der vorliegenden Erfindung ist es, kosmetische Zusammensetzungen bereitzustellen, die Polycarbonsäurederivate enthalten, die nicht toxisch und nicht allergieauslösend sind, wobei diese Polycarbonsäurederivate zum Beispiel als Ersatz für die möglicherweise allergieauslösenden Phthalate eingesetzt werden können.

Diese Aufgabe wird gelöst durch kosmetische Zusammensetzungen enthaltend mindestens ein Cyclohexanpolycarbonsäurederivat.

Der Begriff "kosmetische Zusammensetzung" ist dabei sehr breit zu verstehen. Im Sinne der vorliegenden Anmeldung umfasst der Begriff sowohl Riechstoffzusammensetzungen als auch kosmetische Zusammensetzungen anderer Art wie Reinigungs- und Pflegemittel für Haut und Haar und Zusammensetzungen zur dekorativen Anwendung am menschlichen Körper. Es ist auch möglich, dass eine Riechstoffzusammensetzung enthaltend mindestens ein Cyclohexanpolycarbonsäurederivat zur Herstellung parfumierter Produkte eingesetzt wird, ausgewählt aus Parfum, Eau de Toilette und Ähnlichem, Seife, Duschgel, Badegel, Shampoo, weiteren Haarpflegemitteln, kosmetischen Präparaten wie Cremes und Lotionen oder Sonnenschutzmitteln und des weiteren Körper- und Raumluftdeodorants oder Reinigungsmitteln oder Pflegemitteln für Textilien.

Die Cyclohexanpolycarbonsäurederivate können dabei in den kosmetischen Zusammensetzungen auf vielfältige Weise wirksam sein. Beispielsweise können sie, zum Beispiel in Riechstoffzusammensetzungen, als Lösungsmittel dienen, zum Beispiel als Lösungsmittel für Riechstoffe wie Moschusriechstoffe. Des weiteren können die Cyclohexanpolycarbonsäurederivate zum Beispiel als Weichmacher in Nagellackzusammensetzungen dienen.

Bevorzugt weisen die erfindungsgemäßen kosmetischen Zusammensetzungen mindestens ein Cyclohexanpolycarbonsäurederivat der Formel (I) auf. 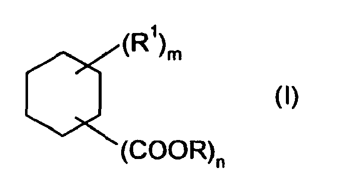 worin
- R¹: für C₁-C₁₀-Alkyl oder C₃-C₈-Cycloalkyl steht,
- m: für 0, 1, 2 oder 3 steht,
- n: für 2, 3 oder 4 steht, und
- R: für Wasserstoff oder iso-Nonyl steht, wobei mindestens ein Rest R für den Vorstehend genannten Alkylrest steht.

Bei den erfindungsgemäß enthaltenen Cyclohexanpolycarbonsäurederivaten handelt es sich insbesondere um Mono-, Di-, Tri-, Tetraester und Anyhdride der Cyclohexanpolycarbonsäuren. Bevorzugt sind alle Carbonsäuregruppen verestert.

Bevorzugt ist das mindestens eine Cyclohexanpolycarbonsäurederivat ausgewählt aus der Gruppe bestehend aus kernhydrierten Mono- und Dialkylestern der Phthalsäure, Isophthalsäure und Terephthalsäure, kernhydrierten Mono-, Di- und Trialkylestern der Trimellitsäure, der Trimesinsäure und der Hemimellitsäure oder Mono-, Di-, Tri- und Tetraalkylestern der Pyrromellitsäure, wobei die Alkylgruppen iso-Nonyl-Gruppen sind.

Im Sinne der vorliegenden Erfindung geeignet sind darüber hinaus auch die in der WO 99/32427 offenbarten, im folgenden nochmals aufgelisteten Cyclohexan-1,2-dicarbonsäureester:
Cyclohexan-1,2-dicarbonsäuredi(isononyl)ester, erhältlich durch Hydrierung eines Di(isononyl)phthalats mit der CAS Nr. 68515-48-0;
Cyclohexan-1,2-dicarbonsäuredi(isononyl)ester, erhältlich durch Hydrierung eines Di(isononyl)phthalats mit der CAS Nr. 28553-12-0, basierend auf n-Buten;
Cyclohexan-1,2-dicarbonsäuredi(isononyl)ester, erhältlich durch Hydrierung eines Di(isononyl)phthalats mit der CAS Nr. 28553-12-0 basierend auf Isobuten;

Des weiteren sind auch die Hydrierungsprodukte der kommerziell erhältlichen Benzolcarbonsäureester mit den Handelsnamen Jayflex DINP (CAS Nr. 68515-48-0), Palatinol 9-P, Vestinol 9 (CAS Nr. 28553-12-0), Palatinol N (CAS Nr. 28553-12-0), als geeignet im Sinne der vorliegenden Erfindung zu bewerten.

Besonders bevorzugte kosmetische Zusammensetzungen umfassen Diester der 1,2 Dicyclohexancarbonsäure. Beispielsweise kann es sich um Di-isononylcyclohexan-1,2-dicarboxylat handeln, welches unter dem Namen Hexamoll® DINCH auch kommerziell erhältlich ist (Fa. BASF AG).

Die Herstellung der Cyclohexanpolycarbonsäurederivate erfolgt bevorzugt gemäß dem in WO 99/32427 offenbarten Verfahren. Dieses Verfahren umfasst die Hydrierung einer Benzolpolycarbonsäure oder eines Derivats davon oder eines Gemisches aus zwei oder mehr davon durch Inkontaktbringen der Benzolpolycarbonsäure oder des Derivats davon oder des Gemischs aus zwei oder mehr davon mit einem Wasserstoff enthaltenden Gas in Gegenwart eines Katalysators der als Aktivmetall mindestens ein Metall der VIII. Nebengruppe des Periodensystems allein oder zusammen mit mindestens einem Metall der I. oder VII. Nebengruppe des Periodensystems, aufgebracht auf einem Träger, umfasst, wobei der Träger Makroporen aufweist.

In einer bevorzugten Ausführungsform weist der Träger einen mittleren Porendurchmesser von mindestens 50 nm und eine BET-Oberfläche von höchstens 30 m²/g auf und die Menge des Aktivmetalls beträgt 0,01 bis 30 Gew.-%, bezogen auf das Gesamtgewicht des Katalysators.

In einer weiteren Ausführungsform wird ein Katalysator eingesetzt, worin die Menge des Aktivmetalls 0,01 bis 30 Gew.-%, bezogen auf das Gesamtgewicht des Katalysators, beträgt und 10 bis 50% des Porenvolumens des Trägers von Makroporen mit einem Porendurchmesser im Bereich von 50 nm bis 10.000 nm und 50 bis 90% des Porenvolumens des Trägers von Mesoporen mit einem Porendurchmesser im Bereich von 2 bis 50 nm gebildet werden, wobei sich die Summe der Anteile der Porenvolumina zu 100% addiert.

In einer weiteren Ausführungsform weist der Katalysator 0,01 bis 30 Gew.%, bezogen auf das Gesamtgewicht des Katalysators, eines Aktivmetalls auf, aufgebracht auf einem Träger, wobei der Träger einen mittleren Porenduchmesser von mindestens 0,1 µm und eine BET-Oberfläche von höchstens 15 m²/g aufweist. Als Träger können prinzipiell alle Träger eingesetzt werden, die Makroporen aufweisen, d.h. Träger, die ausschließlich Makroporen aufweisen, sowie solche, die neben Makroporen auch Meso- und/oder Mikroporen enthalten.

Als Aktivmetall können prinzipiell alle Metalle der VIII. Nebengruppe des Periodensystems eingesetzt werden. Bevorzugt werden als Aktivmetalle Platin, Rhodium, Palladium, Cobalt, Nickel oder Ruthenium oder ein Gemisch aus zwei oder mehr davon eingesetzt, wobei insbesondere Ruthenium als Aktivmetall verwendet wird. Unter den ebenfalls verwendbaren Metallen der I. oder VII. oder aber der I. und der VII. Nebengruppe des Periodensystems, die ebenfalls allesamt prinzipiell verwendbar sind, werden vorzugsweise Kupfer und/oder Rhenium eingesetzt.

Die Begriffe "Makroporen" und "Mesoporen" werden im Rahmen der vorliegenden Anmeldung so verwendet, wie sie in Pure Appl. Chem., 45 S. 79 (1976) definiert sind, nämlich als Poren, deren Durchmesser oberhalb von 50 nm (Makroporen) oder deren Durchmesser zwischen 2 nm und 50 nm liegt (Mesoporen).

Der Gehalt des Aktivmetalls beträgt im allgemeinen 0,01 bis 30 Gew.-%, bevorzugt 0,01 bis 5 Gew.-%, besonders bevorzugt 0,1 bis 5 Gew.%, jeweils bezogen auf das Gesamtgewicht des verwendeten Katalysators.

Der verwendete Begriff "Benzolpolycarbonsäure oder eines Derivats davon" umfasst alle Benzolpolycarbonsäuren an sich, zum Beispiel Phthalsäure, Isophthalsäure, Terephthalsäure, Trimellitsäure, Trimesinsäure, Hemimellitsäure und Pyrromellitsäure und Derivate davon, wobei insbesondere Mono-, Di-, Tri- und Tetraester, insbesondere Alkylester, und Anhydride zu nennen sind.

Bevorzugte Ausführungsformen der geeigneten Katalysatoren sowie bevorzugt eingesetzte Verbindungen und Reaktionsbedingungen sind in WO 99/32427 offenbart.

Ein weiterer Gegenstand der vorliegenden Anmeldung sind kosmetische Zusammensetzungen enthaltend ein Cyclohexanpolycarbonsäurederivat erhältlich gemäß dem vorstehend genannten Verfahren, d.h. die Cyclohexanpolycarbonsäurederivate sind erhältlich durch Hydrierung einer Benzolpolycarbonsäure oder eines Derivats davon oder eines Gemischs aus zwei oder mehr davon durch Inkontaktbringen der Benzolpolycarbonsäure oder des Derivats davon oder des Gemischs aus zwei oder mehr davon mit einem wasserstoffenthaltenden Gas in Gegenwart eines Katalysators, der als Aktivmetall mindestens ein Metall der VIII. Nebengruppe des Periodensystems alleine oder zusammen mit mindestens einem Metall der I. oder VII. Nebengruppe des Periodensystems, aufgebracht auf einem Träger, umfasst, wobei der Träger Makroporen aufweist.

Bevorzugte Ausführungsformen des Cyclohexanpolycarbonsäurederivats sind bereits vorstehend genannt.

Als kosmetische Zusammensetzungen sind alle üblichen kosmetischen Zusammensetzungen geeignet. Dabei handelt es sich bevorzugt um Riechstoffzusammensetzungen, Zusammensetzungen zur Behandlung und Pflege der Haut wie Seife, Dusch- bzw. Badegel, Cremes, Lotions, Sonnenschutzmittel, Deodorants, Zusammensetzungen zur Reinigung und Pflege der Haare wie Haarshampoo, Spülung, Haargel, Haarfestiger in Form von Flüssigkeiten oder Schäumen und weitere Reinigungs- oder Pflegemittel für die Haare. Des weiteren umfassen die kosmetischen Zusammensetzungen Zusammensetzungen zur dekorativen Anwendung am menschlichen Körper wie kosmetische Stifte, zum Beispiel Lippenstifte, Lippenpflegestifte, Abdeckstifte (Concealer), Wangenrouge (Blusher), Lidschattenstifte, Lippenkonturenstifte, Augenkonturenstifte, Augenbrauenstifte, Korrekturstifte, Sonnenschutzstifte, Anti-Akne-Stifte und vergleichbare Produkte sowie Nagellacke und weitere Produkte zur Nagelpflege.

Dabei ist es möglich, dass die genannten Zusammensetzungen zur Reinigung und Pflege der Haut bzw. zur Reinigung und Pflege der Haare sowie die weiteren genannten Zusammensetzungen die ebenfalls aufgeführten Riechstoffzusammensetzungen enthalten, die wiederum mindestens ein erfindungsgemäß eingesetztes Cyclohexanpolycarbonsäurederivat enthalten. Diese Riechstoffzusammensetzungen können jedoch ebenfalls in Raumluftdeodorants, Reinigungsmitteln oder Pflegemitteln für Textilien vorhanden sein.

In einer bevorzugten Ausführungsform handelt es sich bei den erfindungsgemäßen erfindungsgemäßen Zusammensetzungen um Riechstoffzusammensetzungen. Diese Riechstoffzusammensetzungen enthalten mindestens ein Cyclohexanpolycarbonsäurederivat gemäß der vorliegenden Anmeldung. Bevorzugte und besonders bevorzugte Ausführungsformen der Cyclohexanpolycarbonsäurederivate sind bereits vorstehend genannt.

Die üblicherweise in den kosmetischen Zusammensetzungen enthaltenden Komponenten sind dem Fachmann bekannt. Die bevorzugten Riechstoffzusammensetzungen enthalten bevorzugt neben dem erfindungsgemäß mindestens einen eingesetzten Cyclohexanpolycarbonsäurederivat eine, mehrere oder alle der folgenden Verbindungen:
- a): flüchtige phenolische Verbindungen wie Iso-Amylsalicylat, Benzylsalicylat und Thymianöl-Rot,
- b): ätherische Öle, wie Geraniumöl und Patchouliöl,
- c): Zitrusöle,
- d): Extrakte und polymere Materialien wie Benzoinsiamresinoid, Opoponaxresinoid,
- e): "synthetische" Öle wie Bergamot 37 und 430, Geranium 76 und Pomeramsol 314,
- f): Aldehyde und Ketone wie β-Methylnaphthylketon, p-t-Butyl-α-Methylhydrozimtsäurealdehyd und p-t-Amylcyclohexanon,
- g): policyclische Verbindungen wie Kumarin und β-Naphthylmethylether,
- h): weitere Ester (neben den erfindungsgemäß eingesetzten Cyclohexanpolycarbonsäurederivaten, bevorzugt Cyclohexanpolycarbonsäureestem) wie Diethylphthalat und Phenylethylphenylacetat, wobei bevorzugt keine aromatischen Polyalkylester wie Diethylphthalat eingesetzt werden,
- i): Alkohole wie Dimyrcetol, Phenylethylalkohol und Tetrahydromuguol.

Des weiteren enthalten die Riechstoffzusammensetzungen bevorzugt Ester und ätherische Öle von floralen Materialien und Früchten, Zitrusölen, Aldehyden, Resinoiden, Moschus und anderen tierischen Gerüchen wie natürlichen Isolaten der Cewet-Katze, des Bibergeils und des Moschusochsen.

Ein weiterer Gegenstand der vorliegenden Anmeldung ist ein parfümiertes Produkt ausgewählt aus der Gruppe bestehend aus Parfum, Eau de Toilette, Seife, Duschgel, Badegel, Shampoo, weiteren Haarpflegemitteln, kosmetischen Präparaten, Körperdeodorant, Raumluftdeodorant, Reinigungsmittel für Textilien und Pflegemittel für Textilien, enthaltend eine erfindungsgemäße Riechstoffzusammensetzung. Geeignete parfümierte Produkte sind bereits vorstehend genannt. Bevorzugt wird die erfindungsgemäße Riechstoffzusammensetzung in Körperdeodorants eingesetzt.

Weitere Komponenten der parfümierten Produkte sind dem Fachmann bekannt.

Eine weitere bevorzugte Ausführungsform der erfindungsgemäßen kosmetischen Zusammensetzungen betrifft kosmetische Zusammensetzungen zur dekorativen Anwendung am menschlichen Körper. Bei diesen Zusammensetzungen handelt es sich bevorzugt um Nagellacke bzw. weitere Zusammensetzungen zur Nagelpflege, sowie kosmetische Stifte wie Lippenstifte, Lippenpflegestifte, Abdeckstifte (Concealer), Wangenrouge (Blusher), Lidschattenstifte, Lippenkonturenstifte, Augenkonturenstifte, Augenbrauenstifte, Korrekturstifte, Sonnenschutzstifte, Anti-Akne-Stifte und vergleichbare Produkte. Bevorzugt handelt es sich bei den erfindungsgemäßen Zusammensetzungen um Nagellacke und Lippenstifte.

Geeignete Komponenten der genannten kosmetischen Zusammensetzungen zur dekorativen Anwendung am menschlichen Körper sind dem Fachmann bekannt.

In Nagellacken werden die erfindungsgemäß eingesetzten Cyclohexanpolycarbonsäurederivate bevorzugt als Weichmacher eingesetzt und können so die allergieauslösenden Weichmacher wie Diethylphthalat ersetzen.

Ein weiterer Gegenstand der vorliegenden Anmeldung betrifft die Verwendung mindestens eines Cyclohexanpolycarbonsäurederivats in kosmetischen Zusammensetzungen. Bevorzugte Cyclohexanpolycarbonsäurederivate und kosmetische Zusammensetzungen sind bereits vorstehend genannt. Der Einsatz solcher Cyclohexanpolycarbonsäurederivate in kosmetischen Zusammensetzungen ist bisher nicht bekannt. Die Cyclohexanpolycarbonsäurederivate können verschiedene Aufgaben in den erfindungsgemäßen kosmetischen Zusammensetzungen erfüllen. Bei Verwendung in Riechstoffzusammensetzungen können die erfindungsgemäß eingesetzten Cyclohexanpolycarbonsäurederivate als Lösungsmittel dienen, in Nagellacken sind die erfindungsgemäß eingesetzten Cyclohexanpolycarbonsäurederivate als Weichmacher geeignet. Wesentlich ist, dass die erfindungsgemäßen Cyclohexanpolycarbonsäurederivate toxikologisch unbedenklich sind und eine hervorragende Lösekraft aufweisen. Des weiteren können die erfindungsgemäß eingesetzten Cyclohexanpolycarbonsäurederivate eine emulgierende Wirkung aufweisen sowie einen Einfluss auf Gleit- und Hafteigenschaften bei der Verarbeitung zu zum Beispiel kosmetischen Stiften aufweisen.

Bevorzugt werden die erfindungsgemäß eingesetzten Cyclohexanpolycarbonsäurederivate in Riechstoffzusammensetzungen, insbesondere in Deodorants, und in kosmetischen Zusammensetzungen zur dekorativen Anwendung am menschlichen Körper, insbesondere in kosmetischen Stiften und Nagellacken verwendet.

## Patentansprüche

1. Kosmetische Zusammensetzung enthaltend mindestens ein Cyclohexanpolycarbonsäurederivat der Formel (I) 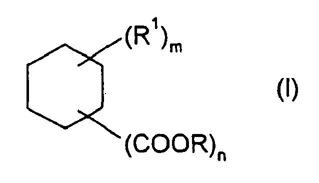 worin
R¹ für C₁-C₁₀-Alkyl oder C₃-C₈-Cycloalkyl steht;
m für 0, 1, 2 oder 3 steht;
n für 2, 3 oder 4 steht, und
R für H oder iso-Nonyl steht, wobei mindestens ein Rest R für den vorstehend genannten Alkylrest steht.

2. Kosmetische Zusammensetzung nach Anspruch 1, **dadurch gekennzeichnet, dass** das mindestens eine Cyclohexanpolycarbonsäurederivat ausgewählt ist aus der Gruppe bestehend aus kernhydrierten Mono- und Dialkylestern der Phthalsäure, Isophthalsäure und Terephthalsäure, kernhydrierten Mono-, Di- und Trialkylestern der Trimellitsäure, der Trimesinsäure und der Hemimillitsäure oder Mono-, Di, Tri- und Tetraalkylestern der Pyrromellitsäure, wobei die Alkylgruppen iso-Nonyl-Gruppen sind.

3. Kosmetische Zusammensetzung nach Anspruch 1 oder 2, **dadurch gekennzeichnet, dass** das mindestens eine Cyclohexanpolycarbonsäurederviat ausgewählt ist aus der Gruppe bestehend aus
Cyclohexan-1,2-dicarbonsäuredi(isononyl)ester, erhältlich durch Hydrierung eines Di(isononyl)phthalats mit der CAS Nr. 68515-48-0;
Cyclohexan-1,2-dicarbonsäuredi(isononyl)ester, erhältlich durch Hydrierung eines Di(isononyl)phthalats mit der CAS Nr. 28553-12-0, basierend auf n-Buten;
Cyclohexan-1,2-dicarbonsäuredi(isononyl)ester, erhältlich durch Hydrierung eines Di(isononyl)phthalats mit der CAS Nr. 28553-12-0 basierend auf Isobuten;
Cydohexan-1,2-dicarbonsäwediisononylester die Hydrierungsprodukte der kommerziell erhältlichen Benzolcarbonsäureester mit den Handelsnamen Jayflex DINP (CAS Nr. 68515-48-0) Palatinol 9-P, Vestinol 9 (CAS Nr. 28553-12-0), Palatinol N (CAS-Nr. 28553-12-0) und Jayflex L9P (CAS Nr. 68515-45-7).

4. Kosmetische Zusammensetzung nach einem der Ansprüche 1 bis 3 oder 13, enthaltend ein Cyclohexanpolycarbonsäurederivat erhältlich durch Hydrierung einer Benzolpolycarbonsäure oder eines Derviats davon oder eines Gemisches aus zwei oder mehr davon durch Inkontaktbringen der Benzolpolycarbonsäure oder des Derivats davon oder des Gemisches aus zwei oder mehr davon mit einem Wasserstoff enthaltenden Gas in Gegenwart eines Katalysators, der als Aktivmetall mindestens ein Metall der VIII. Nebengruppe des Periodensystems allein oder zusammen mit mindestens einem Metall der I. oder VII. Nebengruppe des Periodensystems, aufgebracht auf einen Träger, umfasst, wobei der Träger Makroporen aufweist.

5. Kosmetische Zusammensetzung nach einem der Ansprüche 1 bis 4 oder 13, **dadurch gekennzeichnet, dass** die kosmetische Zusammensetzung eine Riechstoffzusammensetzung ist.

6. Riechstoffzusammensetzung nach Anspruch 5 enthaltend mindestens ein Cyclohexanpolycarbonsäurederivat gemäß einem der Ansprüche 1 bis 4 oder 13 sowie eine, mehrere oder alle der nachfolgend genannten Verbindungen:
a) flüchtige phenolische Verbindungen ,
b) ätherische Öle,
c) Zitrusöle,
d) Extrakte und polymere Materialien,
e) "synthetische" Öle
f) Aldehyde und Ketone,
g) polycyclische Verbindungen,
h) weitere Ester, und
i) Alkohole.

7. Parfümiertes Produkt ausgewählt aus der Gruppe bestehend aus Parfum, Eau de Toilette, Seife, Duschgel, Badegel, Shampoo, weiteren Haarpflegemitteln, kosmetischen Präparaten, Körperdeodorant, Raumluftdeodorant, Reinigungsmittel für Textilien und Pflegemittel für Textilien enthaltend eine Riechstoffzusammensetzung nach Anspruch 6.

8. Kosmetische Zusammensetzung nach einem der Ansprüche 1 bis 4 oder 13, **dadurch gekennzeichnet, dass** die kosmetische Zusammensetzung eine Zusammensetzung zur dekorativen Anwendung am menschlichen Körper ist.

9. Kosmetische Zusammensetzung nach Anspruch 8, **dadurch gekennzeichnet, dass** die kosmetische Zusammensetzung zur dekorativen Anwendung am menschlichen Körper ausgewählt ist aus kosmetischen Stiften und Nagellacken.

10. Verwendung von mindestens einem Cyclohexanpolycarbonsäurederivat gemäß einem der Ansprüche 1 bis 4 oder 13 in kosmetischen Zusammensetzungen.

11. Verwendung von mindestens einem Cyclohexanpolycarbonsäurederivat gemäß einem der Ansprüche 1 bis 4 oder 13 als Lösungsmittel in Riechstoffzusammensetzungen.

12. Verwendung von mindestens einem Cyclohexanpolycarbonsäurederivat gemäß einem der Ansprüche 1 bis 4 oder 13 in kosmetischen Zusammensetzungen zur dekorativen Anwendung am menschlichen Körper, insbesondere in kosmetischen Stiften und Nagellacken.

13. Kosmetische Zusammensetzung nach einem der Ansprüche 1 bis 4, **dadurch gekennzeichnet, dass** das Cyclohexanpolycarbonsäurederivat der Formel (I) Diisononylcyclohexan-1,2-dicarboxylat ist.

## Claims

1. A cosmetic composition comprising at least one cyclohexanepolycarboxylic acid derivative of the formula (I) 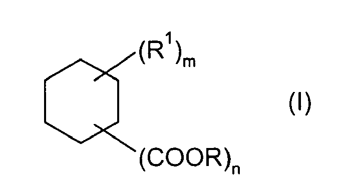 in which
R¹ is C₁-C₁₀-alkyl or C₃-C₈-cycloalkyl ;
m is 0, 1, 2 or 3;
n is 2, 3 or 4, and
R is H or, isononyl, where at least one radical R is the abovementioned alkyl radical.

2. The cosmetic composition according to claim 1, wherein the at least one cyclohexanepolycarboxylic acid derivative is chosen from the group consisting of ring-hydrogenated mono- and dialkyl esters of phthalic acid, isophthalic acid and terephthalic acid, ring-hydrogenated mono-, di- and trialkyl esters of trimellitic acid, of trimesic acid and of hemimillitic acid, or mono-, di, tri- and tetraalkyl esters of pyrromellitic acid, where the alkyl groups are isononyl groups.

3. The cosmetic composition according to claim 1 or 2, wherein the at least one cyclohexanepolycarboxylic acid derivative is chosen from the group consisting of
di(isononyl) esters of cyclohexane-1,2-dicarboxylic acid obtainable via hydrogenation of a di(isononyl) phthalate with the CAS No. 68515-48-0;
di(isononyl) esters of cyclohexane-1,2-dicarboxylic acid obtainable via hydrogenation of a di(isononyl) phthalate with the CAS No. 28553-12-0, based on n-butene;
di(isononyl) esters of cyclohexane-1,2-dicarboxylic acid obtainable via hydrogenation of a di(isononyl) phthalate with the CAS No. 28553-12-0, based on isobutene;
- diisononyl ester of cyclohexane-1,2-dicarboxylic acid, the hydrogenation products of the commercially available benzenecarboxylic esters with the trade names Jayflex DINP (CAS No. 68515-48-0), Palatinol 9-P, Vestinol 9 (CAS No. 28553-12-0), Palatinol N (CAS No. 28553-12-0) and Jayflex L9P (CAS No. 68515-45-7).

4. The cosmetic composition according to any of claims 1 to 3 or 13, comprising a cyclohexanepolycarboxylic acid derivative obtainable via hydrogenation of a benzenepolycarboxylic acid or of a derivative thereof, or via hydrogenation of a mixture of two or more thereof, by bringing the benzenepolycarboxylic acid or the derivative thereof or the mixture of two or more thereof into contact with a hydrogen-comprising gas in the presence of a catalyst which comprises, as active metal, at least one metal of the 8th transition group of the Periodic Table of the Elements alone or together with at least one metal of the 1st or 7th transition group of the Periodic Table of the Elements, applied to a support which has macropores.

5. The cosmetic composition according to any of claims 1 to 4 or 13, wherein the cosmetic composition is a fragrance composition.

6. The fragrance composition according to claim 5 comprising at least one cyclohexanepolycarboxylic acid derivative according to any of claims 1 to 4 or 13, and one, some or all of the compounds specified below:
a) volatile phenolic compounds,
b) essential oils,
c) citrus oils,
d) extracts and polymeric materials,
e) "synthetic" oils
f) aldehydes and ketones;
g) polycyclic compounds,
h) further esters, and
i) alcohols.

7. A perfumed product chosen from the group consisting of perfume, Eau de Toilette, soap, shower gel, bath gel, shampoo, further haircare compositions, cosmetic preparations, body deodorant, room deodorant, detergents for textiles and care compositions for textiles comprising a fragrance composition according to claim 6.

8. The cosmetic composition according to any of claims 1 to 4 or 13, wherein the cosmetic composition is a composition for decorative application on the human body.

9. The cosmetic composition according to claim 8, wherein the cosmetic composition for decorative application on the human body is chosen from cosmetic sticks and nail varnishes.

10. The use of at least one cyclohexanepolycarboxylic acid derivative according to any of claims 1 to 4 or 13 in cosmetic compositions.

11. The use of at least one cyclohexanepolycarboxylic acid derivative according to any of claims 1 to 4 or 13 as a solvent in fragrance compositions.

12. The use of at least one cyclohexanepolycarboxylic acid derivative according to any of claims 1 to 4 or 13 in cosmetic compositions for decorative application on the human body, in particular in cosmetic sticks and nail varnishes.

13. A cosmetic composition according to any of claims 1 to 4, wherein the cyclohexanepolycarboxylic acid derivative of the formula (I) is diisononylcyclohexane 1,2-dicarboxylate.

## Revendications

1. Composition cosmétique contenant au moins un dérivé d'acide cyclohexanepolycarboxylique de formule (I) : 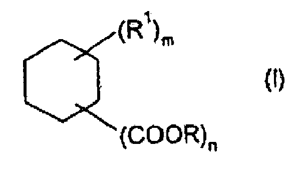 dans laquelle
R¹ désigne un radical C₁-C₁₀-alkyle ou C₃-C₈-cycloalkyle;
m désigne 0, 1, 2 ou 3;
n désigne 2, 3 ou 4; et
R désigne H ou un radical isononyle, au moins un reste R désignant le reste alkyle précité.

2. Composition cosmétique selon la revendication 1, **caractérisée en ce qu'**au moins un dérivé d'acide cyclohexanepolycarboxylique est sélectionné à partir du groupe composé des mono- et dialkylesters de l'acide phthalique, de l'acide isophthalique et de l'acide terephthalique, hydrogénés en leur noyau, des mono-, di- et trialkylesters de l'acide trimellitique, de l'acide trimésinique et de l'acide hémimellitique, hydrogénés en leur noyau, ou des mono-, di-, tri- et tétraalkylesters de l'acide pyromellitique, les groupes alkyle étant des groupes isononyle.

3. Composition cosmétique selon l'une des revendications 1 ou 2, **caractérisée en ce que** l'au moins un dérivé d'acide cyclohexanepolycarboxylique est sélectionné dans le groupe composé du :
di(isononyl)ester d'acide cyclohexane-1,2-dicarboxylique, obtenu par hydrogénation d'un di(isononyl)phthalate portant le n° CAS 68515-48-0;
di(isononyl)ester d'acide cyclohexane-1,2-dicarboxylique, obtenu par hydrogénation d'un di(isononyl)phthalate portant le n° CAS 28553-12-0, basé sur du n-butène;
di(isononyl)ester d'acide cyclohexane-1,2-dicarboxylique, obtenu par hydrogénation d'un di(isononyl)phthalate avec le n° CAS 28553-12-0, basé sur de l'isobutène;
diisononylester d'acide cyclohexane-1,2-dicarboxylique;
les produits d'hydrogénation des esters d'acide benzènecarboxylique obtenus dans le commerce avec les noms commerciaux Jayflex DINP (n° CAS 68515-48-0), Palatinol 9-P, Vestinol 9 (n° CAS 28553-12-0), Palatinol N (n° CAS 28553-12-0) et Jayflex L9P (n° CAS 68515-45-7).

4. Composition cosmétique selon l'une des revendications 1 à 3 ou 13, contenant un dérivé d'acide cyclohexanepolycarboxylique obtenu par hydrogénation d'un acide benzènepolycarboxylique ou d'un dérivé de celui-ci ou d'un mélange de deux ou plusieurs d'entre eux par mise en contact de l'acide benzènepolycarboxylique ou du dérivé de celui-ci ou du mélange de deux ou plusieurs d'entre eux avec un gaz contenant de l'hydrogène en présence d'un catalyseur qui présente comme métal actif au moins un métal du groupe VIII du système périodique seul ou avec au moins un métal des groupes I ou VII du système périodique, appliqué sur un support, le support présentant des macropores.

5. Composition cosmétique selon l'une des revendications 1 à 4 ou 13, **caractérisée en ce que** la composition cosmétique est une composition de parfum.

6. Composition de parfum selon la revendication 5, contenant au moins un dérivé d'acide cyclohexanepolycarboxylique selon l'une des revendications 1 à 4 ou 13 ainsi qu'un, plusieurs ou tous les composés ci-dessous :
a) composés phénoliques volatils;
b) huiles essentielles;
c) huiles d'agrumes;
d) extraits et matériaux polymères;
e) huiles "synthétiques";
f) aldéhydes et cétones;
g) composés polycycliques;
h) autres esters, et
i) alcools.

7. Produit parfumé sélectionné dans le groupe composé de parfum, d'eau de toilette, de savon, de gel douche, de bain douche, de shampooing, d'autres soins capillaires, de préparations cosmétiques, de déodorant corporel, de désodorisant ambiant, de produit lessiviel pour textiles et de soins pour les textiles contenant une composition de parfum selon la revendication 6.

8. Composition cosmétique selon l'une des revendications 1 à 4 ou 13, **caractérisée en ce que** la composition cosmétique est une composition pour une application décorative sur le corps humain.

9. Composition cosmétique selon la revendication 8, **caractérisée en ce que** la composition cosmétique pour l'application décorative sur le corps humain est sélectionnée à partir de rouges à lèvres et vernis à ongles cosmétiques.

10. Utilisation d'au moins un dérivé d'acide cyclohexanepolycarboxylique selon l'une des revendications 1 à 4 ou 13 dans des compositions cosmétiques.

11. Utilisation d'au moins un dérivé d'acide cyclohexanepolycarboxylique selon l'une des revendications 1 à 4 ou 13 comme solvant dans des compositions de parfum.

12. Utilisation d'au moins un dérivé d'acide cyclohexanepolycarboxylique selon l'une des revendications 1 à 4 ou 13 dans des compositions cosmétiques pour l'application décorative sur le corps humain, en particulier dans les rouges à lèvres et vernis à ongles cosmétiques.

13. Composition cosmétique selon l'une des revendications 1 à 4, **caractérisée en ce que** le dérivé d'acide cyclohexanepolycarboxylique de formule (I) est le 1,2-dicarboxylate de diisononylcyclohexane.
